# EUROPÄISCHE PATENTSCHRIFT

(11) **EP 0 918 252 B1**
(45) Veröffentlichungstag und Bekanntmachung des Hinweises auf die Patenterteilung: **13.02.2002**
(21) Anmeldenummer: 98121659.1
(22) Anmeldetag: 12.11.1998
(51) Int. Cl.: G03C 7/407, G03C 5/305, C07C 259/06

(54) **Wässrige Zubereitung als Oxidationsschutzmittel**
Aqueous composition as antioxidant
Composition aqueuse utilisée comme antioxydant

(30) Priorität: 24.11.1997 DE 19751945
(43) Veröffentlichungstag der Anmeldung: 26.05.1999
(73) Patentinhaber: Agfa-Gevaert, 2640 Mortsel (BE)
(72) Erfinder: Odenwälder, Heinrich Dr., 51381 Leverkusen (DE); Hübsch, Thomas Dr., 51061 Köln (DE); Scholkmann, Angelika, 51373 Leverkusen (DE); Dovecar, Frank, 51377 Leverkusen (DE)

(56) Entgegenhaltungen:
- EP-A- 0 148 432
- US-A- 3 609 163
- US-A- 4 055 426
- PATENT ABSTRACTS OF JAPAN vol. 018, no. 228 (P-1730), 25. April 1994 & JP 06 019093 A (KONICA CORP), 28. Januar 1994

## Beschreibung

Die Erfindung betrifft die wäßrige Zubereitung bestimmter organischer Verbindungen, die als Oxidationsschutz verwendet werden kann.

Bestimmte technische Produkte, wie fotografische Entwickler, die oxidationsempfindlich sind, werden mit Oxidationsschutzmitteln versehen, die ein Oxidieren der Entwicklersubstanz verhindern oder zumindest vermindern sollen.

Aus JP-A-06 019 093 sind Oxidationsschutzmittel in festen Entwicklerzusammensetzungen bekannt, wobei u. a. Hydrowamsäurederivate genannt werden.
US-A-4,055,426 beansprucht dagegen bestimmte mindergiftige Hydroxamsäuren als Oxidationsschutz für Entwicklerlösungen. In US-A-3,609,163 wird die Herstellung wässriger Lösungen von Hydroxamsäuren als Zwischenschritt einer Synthese ethylenisch ungesättigter cyclischer Nitrilcarbonate beschrieben.

Insbesondere für farbfotografisches Papier zur Herstellung von Farbabzügen, dessen Silberhalogenidemulsionen im wesentlichen aus Silberchlorid bestehen, haben sich als Oxidationsschutzmittel Dialkylhydroxylamine, z.B. Diethylhydroxylamin als besonders wirksam erwiesen, weil sie einerseits die Entwicklersubstanz gegen Oxidation schützen, andererseits aber die Entwicklung, die in höchstens 45 Sekunden abgeschlossen sein muß, nicht hemmen.

Dialkylhydroxylamine haben aber den Nachteil, daß sie sehr unangenehm riechen. Dieses Problem hat sich in jüngster Zeit noch verstärkt, weil die Entwickler-Regenerate immer konzentrierter angeboten werden und somit eine immer höhere Konzentration an den unangenehm riechenden Dialkylhydroxylaminverbindungen enthalten.

Dies ist besonders störend, wenn die Verarbeitungsanlagen für den sogenannten Stundendienst (Minilabs) in Ladengeschäften stehen. Bissulfoethylhydroxylamin, das als Ersatz des Dialkylhydroxylamins verwendet wird, ist zwar geruchlos, aber aufwendig in der Herstellung und verursacht daher erhöhte Kosten.

Aufgabe war daher, geruchslose Oxidationsschutzmittel zu finden, deren Wirksamkeit wenigstens der der Dialkylhydroxylamine entspricht und die wirtschaftlich hergestellt werden können.

Es wurde nun gefunden, daß dies mit bestimmten Hydroxamsäuren gelingt.

Gegenstand der Erfindung sind daher als farbfotografische Entwicklerlösungen geeignete wäßrige Zubereitungen mit wenigstens 20 Gew.-% einer wasserlöslichen Verbindung der Formel (I) 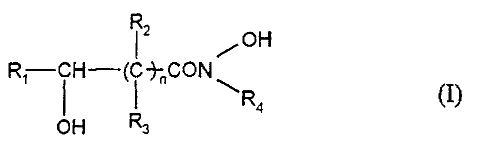 worin
- R₁: ein Wasserstoffatom oder eine Alkylgruppe,
- R₂: ein Wasserstoffatom, eine Alkyl- oder Hydroxylgruppe,
- R₃: ein Wasserstoffatom oder eine Alkylgruppe,
- R₄: ein Wasserstoffatom oder eine Alkylgruppe und
- n: eine Zahl 1 bis 4 bedeuten.

Geeignete Verbindungen sind:

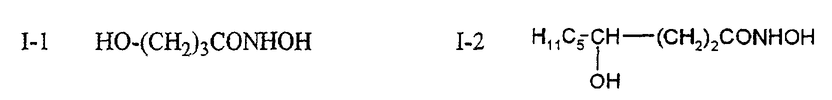

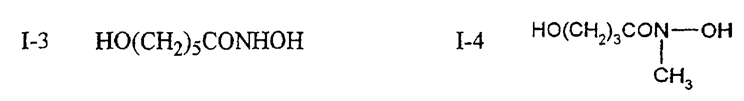

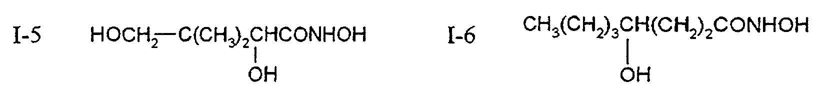

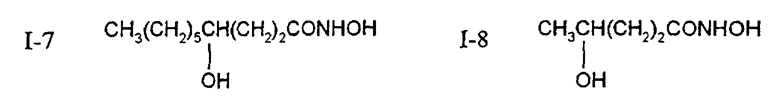

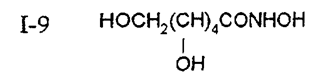

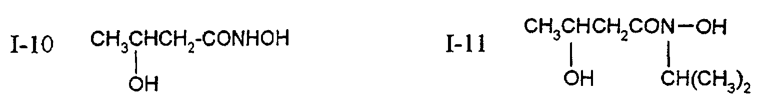

Besonders bevorzugt ist R₄ bzw. ein Wasserstoffatom.

Die Herstellung einer Verbindung der Formel (I) wird an der Verbindung I-1 demonstriert:

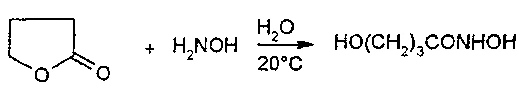

Zu einer Mischung aus 60 g Wasser und 60 g einer 50 gew.-%igen Hydroxylaminlösung werden unter Rühren 86 g γ-Butyrolacton innerhalb von 20 Minuten zugetropft. Durch Zugabe von Wasser bis zu einem Gesamtgewicht von 238 g erhält man eine gebrauchsfertige Lösung von I-1 mit einem Gehalt von 50 Gew.-%.

Verbindungen, für die entsprechende Lactone nicht zur Verfügung stehen, können über geeignete Säurechloride, Säureanhydride oder Ester hergestellt werden.

Die fotografischen Entwickler enthalten darüberhinaus die üblichen Bestandteile, wie Entwicklersubstanz, gegebenenfalls Entwickler-Hilfslösungsmittel, Puffersubstanzen, Alkali und Kalkschutzmittel und sind auf einen pH-Wert zwischen 10 und 11 eingestellt.

Einzelheiten zur Entwicklung und zu den dafür benötigten Chemikalien sind in Research Disclosure (RD) 37254, Teil 10 (1995), S. 294, in RD 37038, Teile XVI bis XXIII (1995), S. 95 ff und in RD 38957, Teile XVIII, XIX und XX (1996), S. 630 ff veröffentlicht.

Insbesondere für die Entwicklung farbfotografischer Materialien sind Entwickler, die eine Verbindung der Formel (I) enthalten, geeignet.

Beispiele für farbfotografische Materialien sind Farbnegativfilme, Farbumkehrfilme, Farbpositivfilme, farbfotografisches Papier, farbumkehrfotografisches Papier, farbempfindliche Materialien für das Farbdiffusionstransfer-Verfahren oder das Silberfarbbleich-Verfahren. Eine Übersicht findet sich in Research Disclosure 37038 (1995) und Research Disclosure 38957 (1996).

Die fotografischen Materialien bestehen aus einem Träger, auf den wenigstens eine lichtempfindliche Silberhalogenidemulsionsschicht aufgebracht ist. Als Träger eignen sich insbesondere dünne Filme und Folien. Eine Übersicht über Trägermaterialien und auf deren Vorder- und Rückseite aufgetragene Hilfsschichten ist in Research Disclosure 37254, Teil 1 (1995), S. 285 und in Research Disclosure 38957, Teil XV (1996), S. 627 dargestellt.

Die farbfotografischen Materialien enthalten üblicherweise mindestens je eine rotempfindliche, grünempfindliche und blauempfindliche Silberhalogenidemulsionsschicht sowie gegebenenfalls Zwischenschichten und Schutzschichten.

Je nach Art des fotografischen Materials können diese Schichten unterschiedlich angeordnet sein. Dies sei für die wichtigsten Produkte dargestellt:

Farbfotografische Filme wie Colornegativfilme und Colorumkehrfilme weisen in der nachfolgend angegebenen Reihenfolge auf dem Träger 2 oder 3 rotempfindliche, blaugrünkuppelnde Silberhalogenidemulsionsschichten, 2 oder 3 grünempfindliche, purpurkuppelnde Silberhalogenidemulsionsschichten und 2 oder 3 blauempfindliche, gelbkuppelnde Silberhalogenidemulsionsschichten auf. Die Schichten gleicher spektraler Empfindlichkeit unterscheiden sich in ihrer fotografischen Empfindlichkeit, wobei die weniger empfindlichen Teilschichten in der Regel näher zum Träger angeordnet sind als die höher empfindlichen Teilschichten.

Zwischen den grünempfindlichen und blauempfindlichen Schichten ist üblicherweise eine Gelbfilterschicht angebracht, die blaues Licht daran hindert, in die darunter liegenden Schichten zu gelangen.

Die Möglichkeiten der unterschiedlichen Schichtanordnungen und ihre Auswirkungen auf die fotografischen Eigenschaften werden in J. Inf Rec. Mats., 1994, Vol. 22, Seiten 183 - 193 und in Research Disclosure 38957 Teil XI (1996), S. 624 beschrieben.

Farbfotografisches Papier, das in der Regel wesentlich weniger lichtempfindlich ist als ein farbfotografischer Film, weist in der nachfolgend angegebenen Reihenfolge auf dem Träger üblicherweise je eine blauempfindliche, gelbkuppelnde Silberhalogenidemulsionsschicht, eine grünempfindliche, purpurkuppelnde Silberhalogenidemulsionsschicht und eine rotempfindliche, blaugrünkuppelnde Silberhalogenidemulsionsschicht auf; die Gelbfilterschicht kann entfallen.

Abweichungen von Zahl und Anordnung der lichtempfindlichen Schichten können zur Erzielung bestimmter Ergebnisse vorgenommen werden. Zum Beispiel können alle hochempfindlichen Schichten zu einem Schichtpaket und alle niedrigempfindlichen Schichten zu einem anderen Schichtpaket in einem fotografischen Film zusammengefaßt sein, um die Empfindlichkeit zu steigern (DE-25 30 645).

Wesentliche Bestandteile der fotografischen Emulsionsschichten sind Bindemittel, Silberhalogenidkörner und Farbkuppler.

Angaben über geeignete Bindemittel finden sich in Research Disclosure 37254, Teil 2 (1995), S. 286 und in Research Disclosure 38957, Teil IIA(1996), S. 598.

Angaben über geeignete Silberhalogenidemulsionen, ihre Herstellung, Reifung, Stabilisierung und spektrale Sensibilisierung einschließlich geeigneter Spektralsensibilisatoren finden sich in Research Disclosure 37254, Teil 3 (1995), S. 286, in Research Disclosure 37038, Teil XV (1995), S. 89 und in Research Disclosure 38957, Teil VA (1996), S. 603.

Fotografische Materialien mit Kameraempfindlichkeit enthalten üblicherweise Silberbromidiodidemulsionen, die gegebenenfalls auch geringe Anteile Silberchlorid enthalten können. Fotografische Kopiermaterialien enthalten entweder Silberchloridbromidemulsionen mit bis 80 mol-% AgBr oder Silberchloridbromidemulsionen mit über 95 mol-% AgCl.

Angaben zu den Farbkupplern finden sich in Research Disclosure 37254, Teil 4 (1995), S. 288, in Research Disclosure 37038, Teil II (1995), S. 80 und in Research Disclosure 38957, Teil XB (1996), S. 616. Die maximale Absorption der aus den Kupplern und dem Farbentwickleroxidationsprodukt gebildeten Farbstoffe liegt vorzugsweise in den folgenden Bereichen: Gelbkuppler 430 bis 460 nm, Purpurkuppler 540 bis 560 nm, Blaugrünkuppler 630 bis 700 nm.

In farbfotografischen Filmen werden zur Verbesserung von Empfindlichkeit, Körnigkeit, Schärfe und Farbtrennung häufig Verbindungen eingesetzt, die bei der Reaktion mit dem Entwickleroxidationsprodukt Verbindungen freisetzen, die fotografisch wirksam sind, z.B. DIR-Kuppler, die einen Entwicklungsinhibitor abspalten.

Angaben zu solchen Verbindungen, insbesondere Kupplern, finden sich in Research Disclosure 37254, Teil 5 (1995), S. 290, in Research Disclosure 37038, Teil XIV (1995), S. 86 und in Research Disclosure 38957, Teil XC (1996), S. 618.

Die meist hydrophoben Farbkuppler, aber auch andere hydrophobe Bestandteile der Schichten, werden üblicherweise in hochsiedenden organischen Lösungsmitteln gelöst oder dispergiert. Diese Lösungen oder Dispersionen werden dann in einer wäßrigen Bindemittellösung (üblicherweise Gelatinelösung) emulgiert und liegen nach dem Trocknen der Schichten als feine Tröpfchen (0,05 bis 0,8 µm Durchmesser) in den Schichten vor.

Geeignete hochsiedende organische Lösungsmittel, Methoden zur Einbringung in die Schichten eines fotografischen Materials und weitere Methoden, chemische Verbindungen in fotografische Schichten einzubringen, finden sich in Research Disclosure 37254, Teil 6 (1995), S. 292.

Die in der Regel zwischen Schichten unterschiedlicher Spektralempfindlichkeit angeordneten nicht lichtempfindlichen Zwischenschichten können Mittel enthalten, die eine unerwünschte Diffusion von Entwickleroxidationsprodukten aus einer lichtempfindlichen in eine andere lichtempfindliche Schicht mit unterschiedlicher spektraler Sensibilisierung verhindern.

Geeignete Verbindungen (Weißkuppler, Scavenger oder EOP-Fänger) finden sich in Research Disclosure 37254, Teil 7 (1995), S. 292, in Research Disclosure 37038, Teil III (1995), S. 84 und in Research Disclosure 38957, Teil XD (1996), S. 621.

Das fotografische Material kann weiterhin UV-Licht absorbierende Verbindungen, Weißtöner, Abstandshalter, Filterfarbstoffe, Formalinfänger, Lichtschutzmittel, Antioxidantien, D_{Min}-Farbstoffe, Zusätze zur Verbesserung der Farbstoff-, Kuppler- und Weißenstabilität sowie zur Verringerung des Farbschleiers, Weichmacher (Latices), Biocide und anderes enthalten.

Geeignete Verbindungen finden sich in Research Disclosure 37254, Teil 8 (1995), S. 292, in Research Disclosure 37038, Teile IV, V, VI, VII, X, XI und XIII (1995), S. 84 ff und in Research Disclosure 38957, Teile VI, VIII, IX und X (1996), S. 607 und 610 ff.

Die Schichten farbfotografischer Materialien werden üblicherweise gehärtet, d.h., das verwendete Bindemittel, vorzugsweise Gelatine, wird durch geeignete chemische Verfahren vernetzt.

Geeignete Härtersubstanzen finden sich in Research Disclosure 37254, Teil 9 (1995), S. 294, in Research Disclosure 37038, Teil XII (1995), Seite 86 und in Research Disclosure 38957, Teil IIB (1996), S. 599.

### Beispiele

### Beispiel 1

Ein farbfotografisches Aufzeichnungsmaterial wurde hergestellt, indem auf einen Schichtträger aus beidseitig mit Polyethylen beschichtetem Papier die folgenden Schichten in der angegebenen Reihenfolge aufgetragen wurden. Die Mengenangaben beziehen sich jeweils auf 1 m². Für den Silberhalogenidauftrag werden die entsprechenden Mengen AgNO₃ angegeben.

### Schichtaufbau 1

| | |
|---|---|
| Schicht 1 | (Substratschicht) |
| | 0,10 g Gelatine |
| | |
| Schicht 2 | (blauempfindliche Schicht): |
| | blauempfindliche Silberhalogenidemulsion (99,5 mol-% Chlorid, 0,5 mol-% Bromid, mittlerer Korndurchmesser 0,9 µm) aus 0,50 g AgNO₃, mit |
| | |
| | 1,25 g Gelatine |
| | 0,42 g Gelkuppler Y-1 |
| | 0,18 g Gelbkuppler Y-2 |
| | 0,50 g Ölbildner OF-1 |
| | 0,10 g Stabilisator ST-1 |
| | 0,70 mg Blausensibilisator BS-1 |
| | 0,30 mg Stabilisator ST-2 |
| | |
| Schicht 3 | (Zwischenschicht) |
| | 1,10 g Gelatine |
| | 0,06 g EOP-Fänger EF-1 |
| | 0,06 g EOP-Fänger EF-2 |
| | 0,12 g Trikresylphosphat (TKP) |
| | |
| Schicht 4 | (grünempfindliche Schicht) |
| | grünempfindliche Silberhalogenidemulsion (99,5 mol-% Chlorid, 0,5 mol-% Bromid, mittlerer Korndurchmesser 0,47 µm) aus 0,28 g AgNO₃, mit |
| | |
| | 1,0 g Gelatine |
| | 0,21 g Purpurkuppler M-1 |
| | 0,15 g Stabilisator ST-3 |
| | 0,21 g Diisodecylphthalat (DIDP) |
| | 0,21 g Isotridecanol (ITD) |
| | 0,70 mg Grünsensibilisator GS-1 |
| | 0,50 mg Stabilisator ST-4 |
| | |
| Schicht 5 | (UV-Schutzschicht) |
| | |
| | 0,95 g Gelatine |
| | 0,30 g UV-Absorber UV-1 |
| | 0,06 g EOP-Fänger EF-1 |
| | 0,06 g EOP-Fänger EF-2 |
| | 0,15 g Ölbildner OF-2 |
| | 0,15 g TKP |
| | |
| Schicht 6 | (rotempfindliche Schicht) |
| | rotempfindliche Silberhalogenidemulsion (99,5 mol-% Chlorid, 0,5 mol-% Bromid, mittlerer Korndurchmesser 0,5 µm) aus 0,30 g AgNO₃, mit |
| | |
| | 1,0 g Gelatine |
| | 0,46 g Blaugrünkuppler C-1 |
| | 0,46 g TKP |
| | 0,03 mg Rotsensibilisator RS-1 |
| | 0,60 mg Stabilisator ST-5 |
| | 0,30 g UV-Absorber UV-2 |
| | |
| Schicht 7 | (UV-Schutzschicht) |
| | 0,30 g Gelatine |
| | 0,10 g UV-Absorber UV-3 |
| | 0,10 g Ölbildner OF-3 |
| | |
| Schicht 8 | (Schutzschicht) |
| | |
| | 0,90 g Gelatine |
| | 0,05 g Weißtöner WT-1 |
| | 0,07 g Beize (Polyvinylpyrrolidon) |
| | 1,20 mg Siliconöl |
| | 2,50 mg Abstandshalter (Polymethylmethacrylat, mittlere Teilchengröße 0,8 µm) |
| | 0,30 g Härtungsmittel H-1 |

Im Beispiel verwendete Verbindungen:

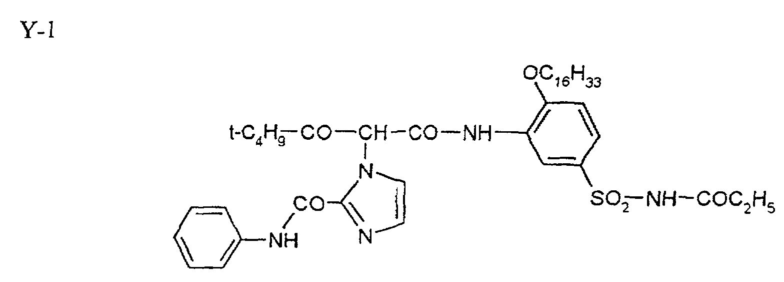

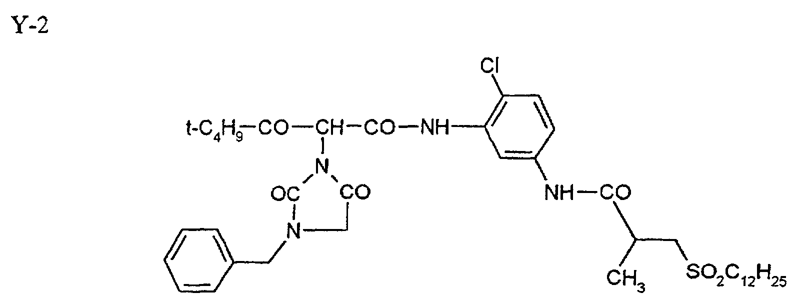

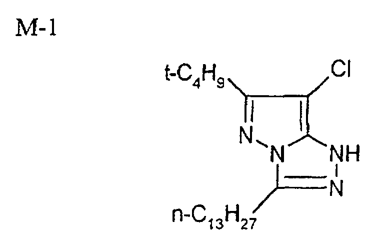

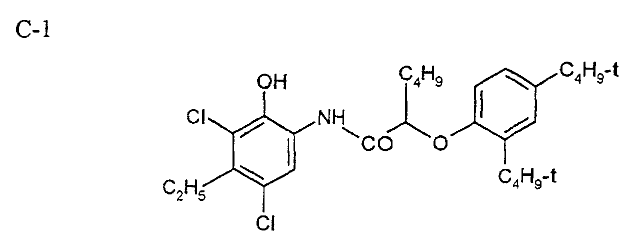

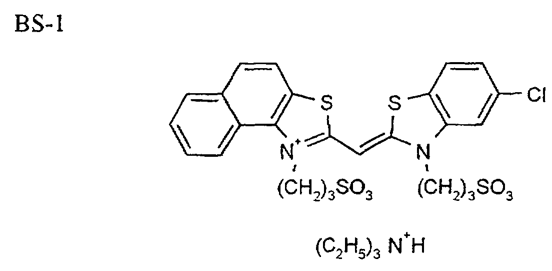

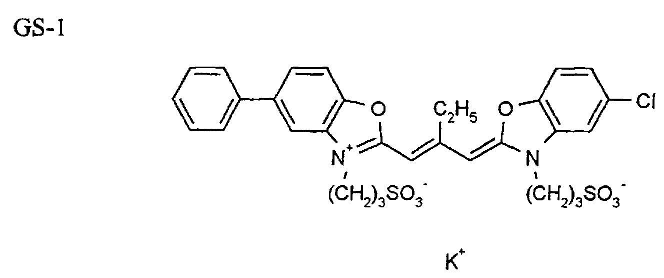

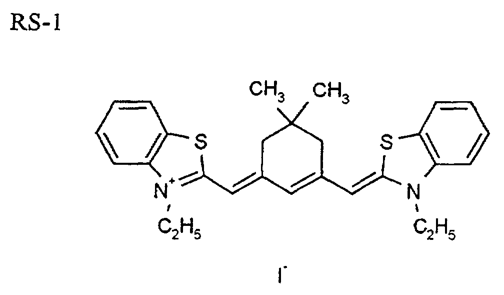

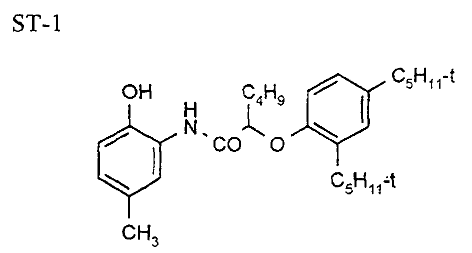

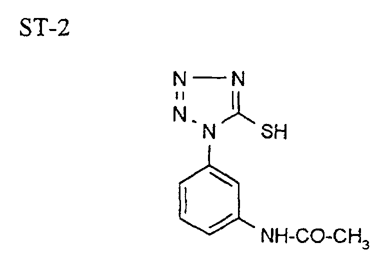

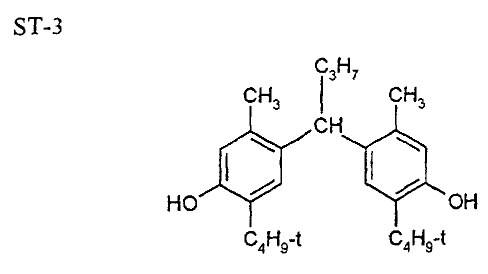

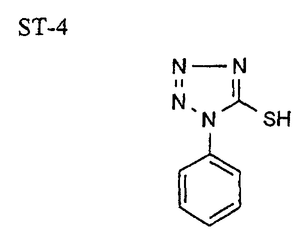

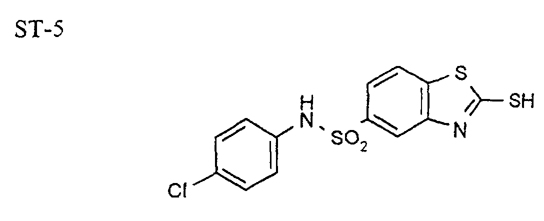

| | | |
|---|---|---|
| OF-1 | Polyester aus HO₂C-(CH₂)₄-CO₂H, HOCH₂-C(CH₃)₂-CH₂OH und HO-C₁₀H₂₁-i | η(20°C):4000-5000 mPa·s n_{D}(20°C): 1.464 - 1.467 |
| | | |
| OF-2 | (̵CH₂―CH₂―CO₂―C₉H₁₉-i)₂ | |
| | | |
| OF-3 | O=P (O-CH₂-CH(C₂H₅)-C₄H₉)₃ | |

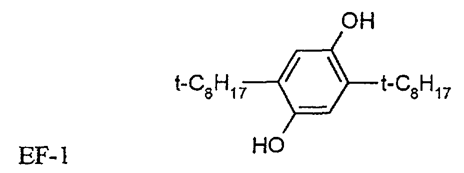

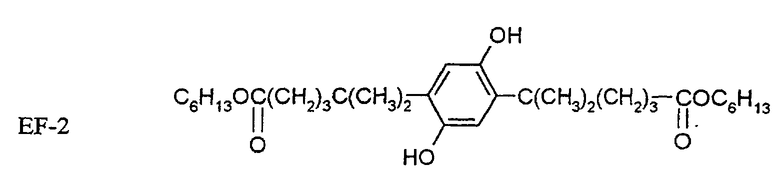

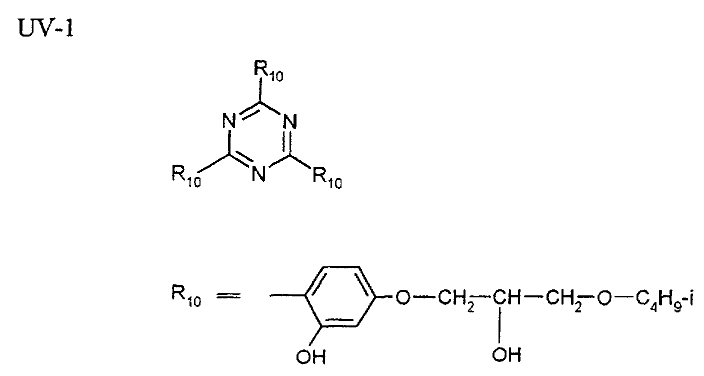

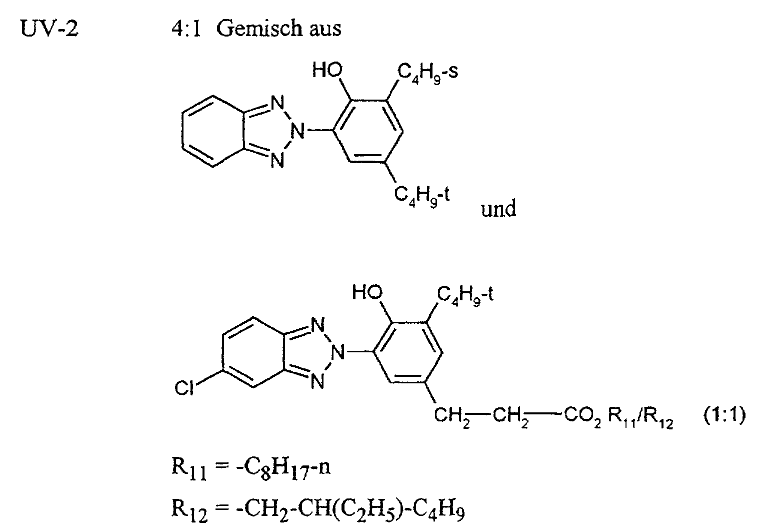

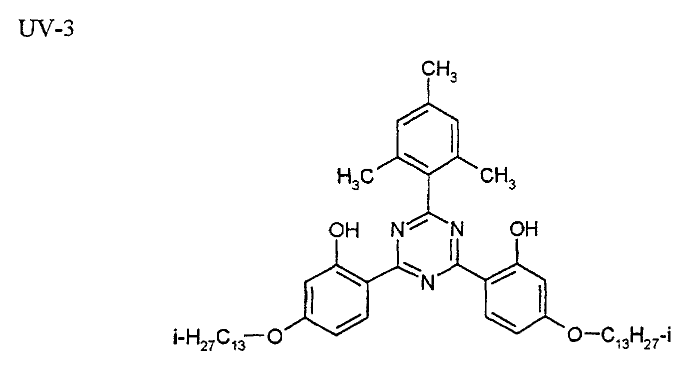

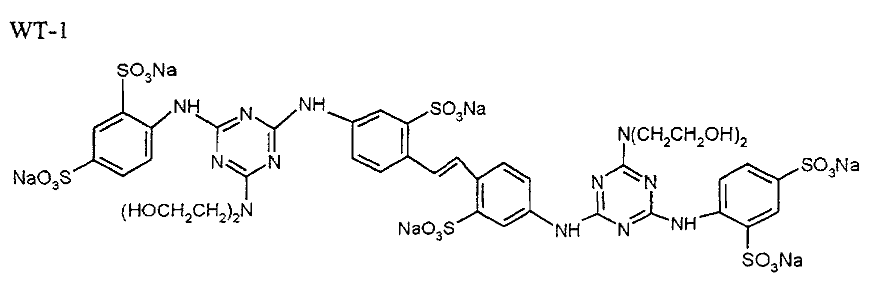

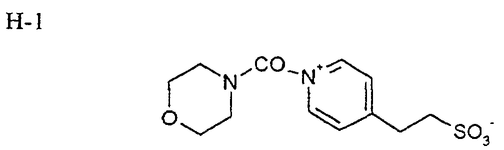

Das Material wurde durch einen Stufenkeil belichtet und anschließend wie folgt verarbeitet:
a) Farbentwickler - 45 s - 38°C

| Farbentwicklerlösung 1 | |
|---|---|
| Caprolactam | 12,0 g |
| N,N-Diethylhydroxylamin | 2,7 g |
| N-Ethyl-N-(2-methansulfonamidoethyl)-4-amino-3-methylbenzolsulfat (CD-3) | 4,8 g |
| Kaliumdisulfit | 0,2 g |
| Kaliumcarbonat | 15,2 g |
| Polymaleinsäureanhydrid | 1,5 g |
| Hydroxyethandiphosphonsäure | 0,1 g |
| Weißtöner (4,4'-Diaminstilbensulfonsäure-Derivat) | 1,5 g |
| Kaliumchlorid | 4,3 g |
| Kaliumhydrogencarbonat | 3,2 g |
| Kaliumbromid | 0,02 g |
| auffüllen mit Wasser auf 1000 ml; pH-Wert mit KOH oder H₂SO₄ auf 10,2 einstellen. | |

b) Bleichfixierbad - 45 s - 35°C

| | |
|---|---|
| Ammonlumthiosulfat | 63,8 g |
| Natriumdisulfit | 16,2 g |
| Ethylendiamintetraessigsäure (Eisen-Ammonium-Salz) | 48,5 g |
| auffüllen mit Wasser auf 1000 ml; pH-Wert mit Ammoniak (25 Gew.-%) oder Essigsäure auf 5,85 einstellen | |

c) Stabilisieren - 90s - 33°C

| | |
|---|---|
| Wasser | 900 ml |
| Natriumdisulfit | 2 g |
| Hydroxyethandiphosphonsäure-dinatriumsalz | 4 g |
| Natriumbenzoat | 0,5 g |
| mit Wasser auf 1000 ml auffüllen. | |

d) Trocknen

Farbentwicklerlösung 2 enthält anstelle von N,N-Diethylhydroxylamin 2,5 g der Verbindung I-1.

Farbentwicklerlösung 3 enthält anstelle von N,N-Diethylhydroxylamin 2,5 g der Verbindung I-8.

In Farbentwicklerlösung 4 wird gegenüber Farbentwicklerlösung 1 N,N-Diethylhydroxylamin weggelassen.

Handelsübliches Colornegativpapier, dessen Emulsionen aus AgCl_{0,995}Br_{0,005} bestehen, wurde durch einen Stufenkeil belichtet. Dabei wurden zusätzliche Filter in den Strahlengang der Belichtungseinheit gebracht, so daß der Keil bei einer optischen Dichte von D = 0.6 neutral erschien.

Das belichtete Colorpapier wurde in den Entwicklerlösungen 1 bis 4 entwickelt, anschließend bleichfixiert, stabilisiert und getrocknet.

Anschließend wurden die Entwickler 1 bis 4 über einen Zeitraum von 3 Wochen in einem abgedeckten Becherglas im Heizschrank bei 40°C gelagert (regelmäßiger Verdunstungsausgleich). In Abständen von je 1 Woche wurde erneut fotografisches Material darin verarbeitet.

Die Ergebnisse der sensitometrischen Auswertung (Gelbschleier Dₘᵢₙ gb und Schultergradation Gelb γ₂gb) sind in Tabellen 1 und 2 aufgeführt.

**Tabelle 1**

| **D**_{**min**}**gb** | **1** | **2** | **3** | **4** |
|---|---|---|---|---|
| frisch | 110 | 114 | 120 | 108 |
| 1 Woche | 126 | 135 | 128 | 270 |
| 2 Wochen | 147 | 147 | 160 | 298 |
| 3 Wochen | 202 | 190 | 201 | - |

**Tabelle 2**

| **γ**_{**2**}**gb** | **1** | **2** | **3** | **4** |
|---|---|---|---|---|
| frisch | 285 | 305 | 290 | 310 |
| 1 Woche | 310 | 297 | 301 | 270 |
| 2 Wochen | 308 | 305 | 336 | 110 |
| 3 Wochen | 319 | 298 | 330 | - |

Es zeigt sich, daß die erfindungsgemäßen Entwickler 2 und 3 eine dem handelsüblichen Entwickler 1 vergleichbare Stabilität besitzen, während ohne Oxidationsschutzmittel (Entwickler 4) nach kurzer Zeit nur noch unbrauchbare Ergebnisse erhalten werden.

### Beispiel 2

In einem Minilab Agfa MSC 101 wurde mit einem Entwicklerregenerator der folgenden Zusammensetzung gearbeitet:

### Farbentwicklerregenerator

| | |
|---|---|
| Caprolactam | 10 g |
| 50 gew.-%ige wäßrige Lösung von I-1 | 5 ml |
| CD-3 | 10 g |
| Kaliumdisulfit | 0,1 g |
| Kaliumcarbonatlösung, 50 gew.-%ig | 23 ml |
| Polymaleinsäureanhydrid, 50 gew.-%ig | 6,7 ml |
| Hydroxyethandiphosphonsäure, 60 gew.-%ig | 0,3 ml |
| Weißtöner (4,4'-Diaminostilbentyp) | 2,4 g |
| Kaliumchlorid | 4,3 g |
| Kaliumhydroxidlösung 50 gew.-%ig | 12,5 ml |
| Kaliumbromid | 0,015 g |
| auffüllen mit Wasser auf 1000 ml, pH-Wert mit KOH auf 11,9 einstellen. | |

In diesem Gerät wurde Colornegativpapier wie in Beispiel 1 unter folgenden Bedingungen verarbeitet:

| Schritt | Zeit | Temperatur | Regenerierquote [ml/m²] |
|---|---|---|---|
| Entwickeln | 33 sec | 38°C | 90 |
| Bleichfixieren | 33 sec | 36°C | 110 |
| Stabilisierbad | 90 sec | 36°C | 250 |

Als Bleichfixier- und Stabilisier-Regeneratoren wurden handelsübliche Produkte verwendet.

Um den Betrieb in einer typischen Umgebung zu simulieren, wurde die Maschine mit wechselndem Durchsatz zwischen 10 und 50 m²/Tag ausgelastet und der Farbentwicklergehalt sowie die Sensitometrie des verarbeiteten Materials bestimmt (Tabelle 3). Aus dem hohen CD 3-Gehalt auch in Phasen sehr geringer Auslastung ergibt sich die hohe Stabilität des erfindungsgemäßen Entwicklerbades.

**Tabelle 3**

| Tag | m² (insg.) | CD 3 [g] | Dₘᵢₙgb | γ₂gb |
|---|---|---|---|---|
| 1 | 0 | 4,9 | 116 | 310 |
| 6 | 120 | 5,7 | - | - |
| 8 | 144 | 5,7 | - | - |
| 15 | 400 | 5,3 | 125 | 297 |
| 20 | 540 | 5,0 | 128 | 305 |
| 26 | 580 | 4,9 | 131 | 315 |

Die sensitometrischen Daten belegen die unter Praxisbedingungen sehr gute Stabilität des Bades.

## Patentansprüche

1. Verwendung einer wässrigen Zubereitung enthaltend wenigstens 20 Gew.-% einer wasserlöslichen Verbindung der Formel (I) 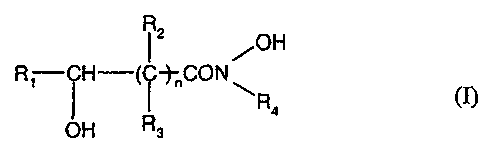 worin
R₁ ein Wasserstoffatom oder eine Alkylgruppe,
R₂ ein Wasserstoffatom, eine Alkyl- oder Hydroxylgruppe,
R₃ ein Wasserstoffatom oder eine Alkylgruppe,
R₄ ein Wasserstoffatom oder eine Alkylgruppe und
n eine Zahl 1 bis 4
bedeuten, als Oxidationsschutzmittel in farbfotografischen Entwicklerlösungen.

2. Farbfotografische Entwicklerlösung, **dadurch gekennzeichnet, daß** sie als Oxidationsschutzmittel eine Verbindung der Formel (I) enthält, wobei
n eine Zahl 2 bis 4
bedeutet.

3. Verfahren zur Verarbeitung eines farbfotografischen, bildmäßig belichteten Silberhalogenidmaterials durch wenigstens die Schritte Farbentwickeln, Bleichen und Fixieren, wobei Bleichen und Fixieren zusammengefaßt sein können, **dadurch gekennzeichnet, daß** eine farbfotografische Entwicklerlösung gemäß Anspruch 2 verwendet wird.

4. Farbfotografische Entwicklerlösung, **dadurch gekennzeichnet, daß** sie als Oxidationsschutzmittel wenigstens eine der Verbindungen 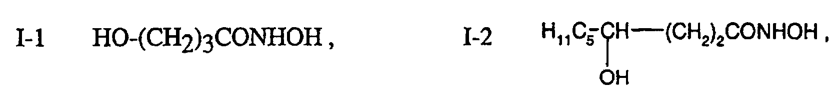 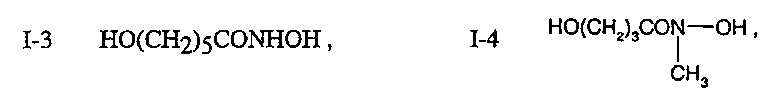 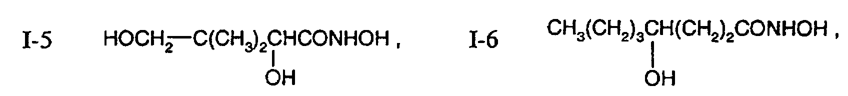 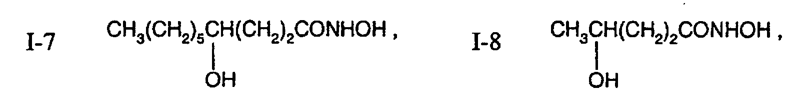 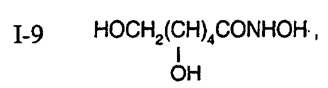 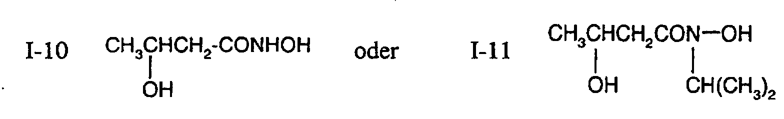 enthält.

5. Farbfotografische Entwicklerlösung nach Anspruch 4, **dadurch gekennzeichnet, daß** sie als Öxidationsschutzmittel wenigstens eine der Verbindungen 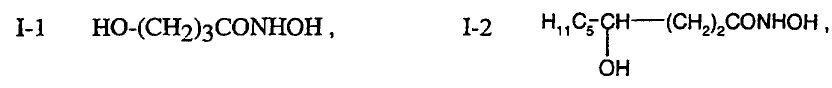 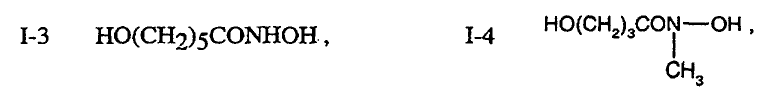 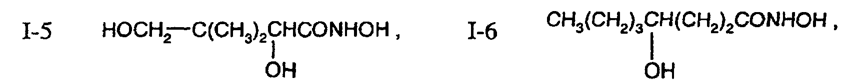 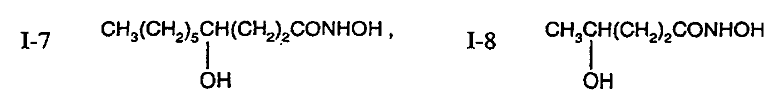 oder 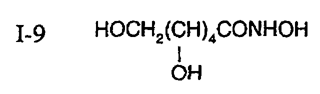 enthält.

6. Verfahren nach Anspruch 3, **dadurch gekennzeichnet, daß** eine farbfotografische Entwicklerlösung nach Anspruch 4 verwendet wird.

7. Farbfotografische Entwickler-Regeneratorlösung, **dadurch gekennzeichnet, daß** sie als Oxidationsschutzmittel eine Verbindung der Formel (I) enthält.

8. Verfahren zur Verarbeitung eines farbfotografischen, bildmäßig belichteten Silberhalogenidmaterials durch wenigstens die Schritte Farbentwickeln, Bleichen und Fixieren, wobei Bleichen und Fixieren zusammengefaßt sein können und wobei verbrauchter Entwickler durch Zudosieren einer Entwickler-Regeneratorlösung nachgeliefert wird, **dadurch gekennzeichnet, daß** eine Entwickler-Regeneratorlösung nach Anspruch 7 verwendet wird.

9. Verfahren nach Anspruch 8, **dadurch gekennzeichnet, daß** die Verarbeitung in einem Minilab erfolgt.

## Claims

1. Use of an aqueous composition as antioxidant in colour photographic developer solutions, the aqueous composition comprising at least 20 % in weight of a water-soluble compound corresponding to the following formula (I): 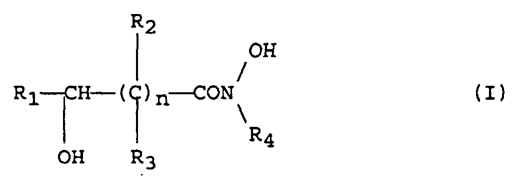 wherein
R₁ represents hydrogen or an alkyl group
R₂ represents hydrogen, an alkyl group or a hydroxyl group
R₃ represents hydrogen or an alkyl group
R₄ represents hydrogen or an alkyl group, and
n is a number from 1 to 4.

2. Colour photographic developer solution, **characterised in that** it comprises as antioxidant a compound corresponding to formula (I), wherein n is a number from 2 to 4.

3. Method of processing an imagewise exposed colour photographic silver halide material comprising at least the steps of colour developing, bleaching and fixing, wherein the bleaching and fixing steps may be combined, **characterised in that** a colour photographic developer solution as defined in claim 2 is used.

4. Colour photographic developer solution, **characterised in that** it comprises as antioxidant at least one of the following compounds :

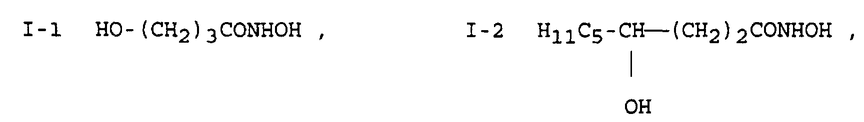

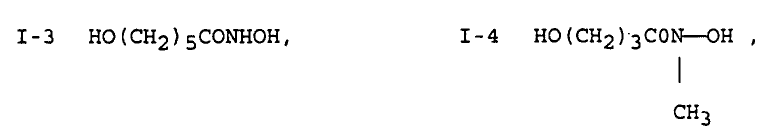

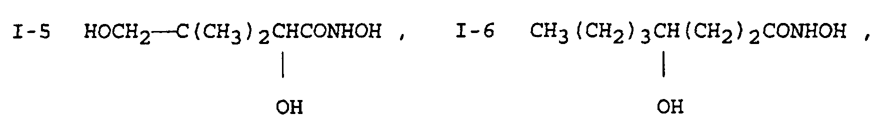

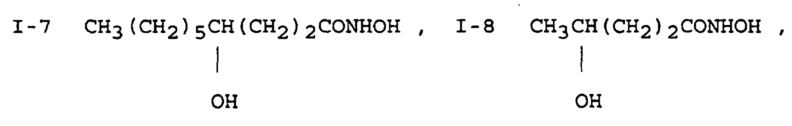

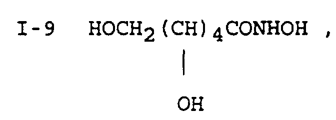

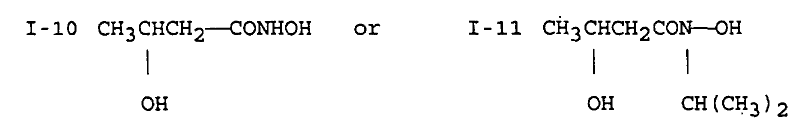

5. Colour photographic developer solution according to claim 4, **characterised in that** it comprises as antioxidant at least one of the following compounds: 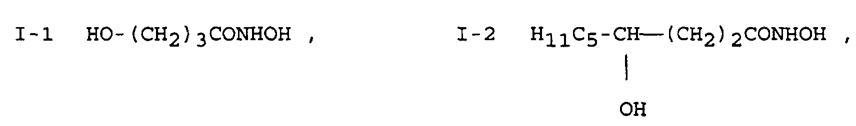 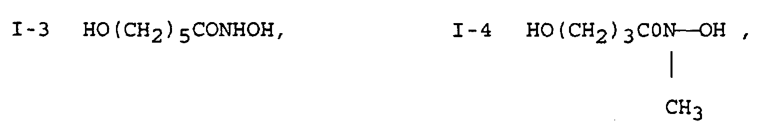 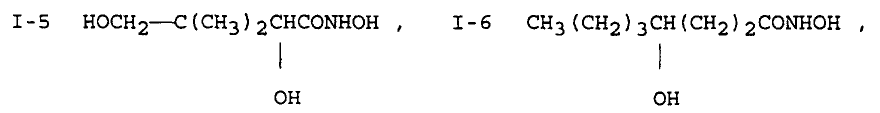 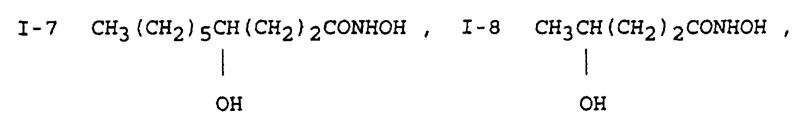 or

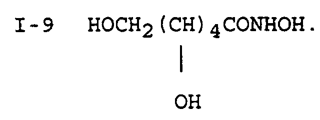

6. Method according to claim 3, **characterised in that** a colour photographic developer solution as defined in claim 4 is used.

7. Colour photographic developer replenisher solution, **characterised in that** it comprises as antioxidant a compound corresponding to the formula (I).

8. Method of processing an imagewise exposed colour photographic silver halide material comprising at least the steps of colour developing, bleaching and fixing, wherein the bleaching and fixing steps may be combined and wherein used developer is replenished by adding a developer replenisher solution, **characterised in that** a developer replenisher solution as defined in claim 7 is used.

9. Method according to claim 8, **characterised in that** the processing is carried out in a minilab.

## Revendications

1. Utilisation d'une composition aqueuse comme antioxydant dans les bains révélateurs photographiques couleur, ladite composition aqueuse contenant au moins 20 % en poids d'un composé soluble dans l'eau répondant à la formule (I) ci-après : 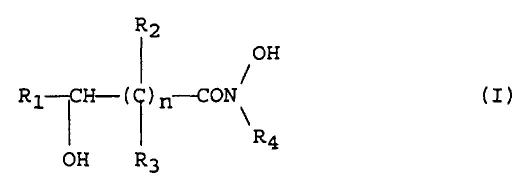 dans laquelle
R₁ représente un atome d'hydrogène ou un groupe alkyle
R₂ représente un atome d'hydrogène, un groupe alkyle ou hydroxyle
R₃ représente un atome d'hydrogène ou un groupe alkyle
R₄ représente un atome d'hydrogène ou un groupe alkyle, et
n est un nombre de 1 à 4.

2. Bain révélateur photographique couleur, **caractérisé en ce qu'**il contient comme antioxydant un composé répondant à la formule (I), dans laquelle n est un nombre de 2 à 4.

3. Procédé de traitement pour un matériau photographique couleur aux halogénures d'argent, exposé suivant une image, comportant au moins les étapes de développement chromogène, blanchiment et fixage, les étapes de blanchiment et fixage pouvant être combinées, **caractérisé en ce qu'**on utilise un bain révélateur photographique couleur tel que défini dans la revendication 2.

4. Bain révélateur photographique couleur, **caractérisé en ce qu'**il contient comme antioxydant au moins un des composés ci-après :

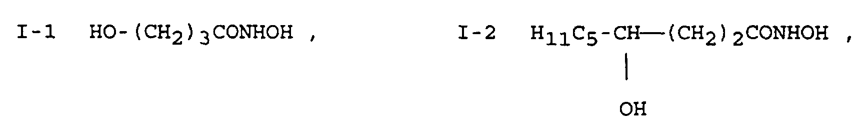

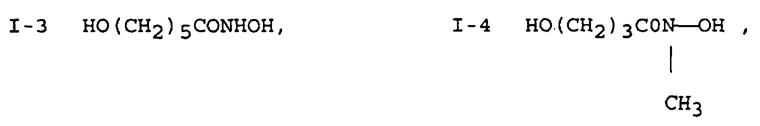

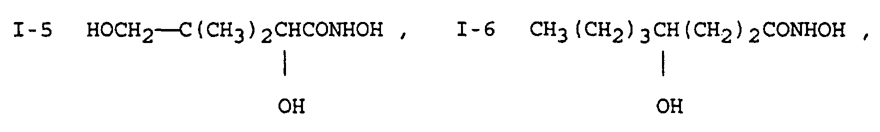

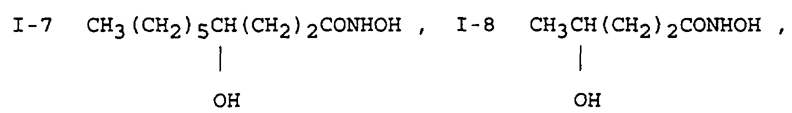

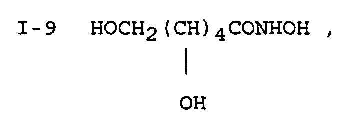

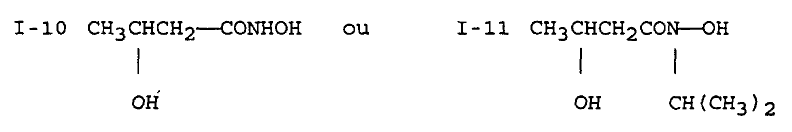

5. Bain révélateur photographique couleur selon la revendication 4, **caractérisé en ce qu'**il contient comme antioxydant au moins un des composés ci-après : 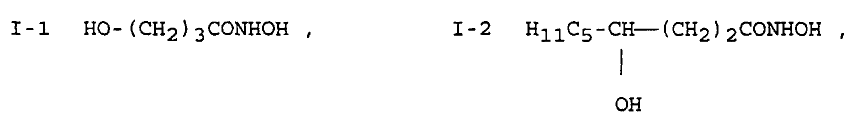 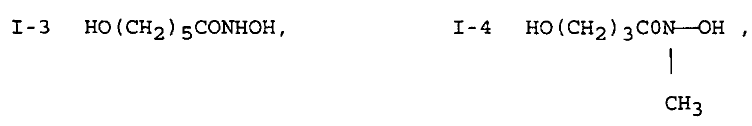 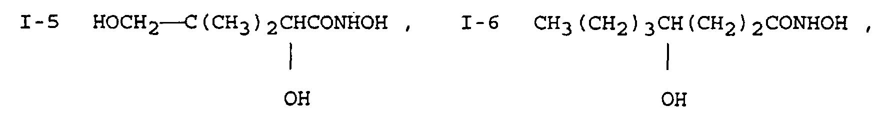 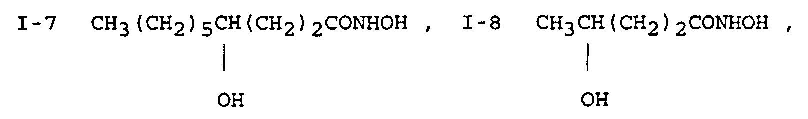 ou

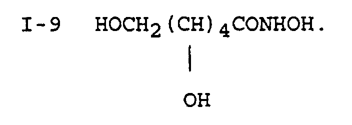

6. Procédé selon la revendication 3, caractérisé en qu'on utilise un bain révélateur photographique couleur tel que défini dans la revendication 4.

7. Solution régénératrice pour bain révélateur photographique couleur, **caractérisée en ce qu'**elle contient comme antioxydant un composé répondant à la formule (I).

8. Procédé de traitement pour un matériau photographique couleur aux halogénures d'argent, exposé suivant une image, comportant au moins les étapes de développement chromogène, blanchiment et fixage, les étapes de blanchiment et fixage pouvant être combinées et le révélateur usagé étant régénéré en l'additionnant d'une solution régénératrice pour révélateur, **caractérisé en ce qu'**on utilise une solution régénératrice pour révélateur telle que définie dans la revendication 7.

9. Procédé selon la revendication 8, **caractérisé en ce que** le traitement est effectué dans un minilab.
